(19) Europäisches Patentamt / European Patent Office / Office européen des brevets

(11) **EP 4 239 060 B1**

(12) **EUROPEAN PATENT SPECIFICATION**

(45) Date of publication and mention
of the grant of the patent:
**20.05.2026 Bulletin 2026/21**

(21) Application number: **21886835.4**

(22) Date of filing: **28.10.2021**

(51) International Patent Classification (IPC):
*A61K 40/42* (2025.01)    *A61K 40/15* (2025.01)
*A61K 40/35* (2025.01)    *A61P 31/00* (2006.01)
*C07K 14/52* (2006.01)    *C07K 14/54* (2006.01)
*C12N 5/0783* (2010.01)    *C12N 15/85* (2006.01)

(52) Cooperative Patent Classification (CPC):
**A61P 31/00; A61K 40/15; A61K 40/35;
A61K 40/42; C07K 14/523; C07K 14/5418;
C12N 5/0636; C12N 5/0646; C12N 15/85;**
C12N 2840/203

(86) International application number:
**PCT/KR2021/015290**

(87) International publication number:
**WO 2022/092841 (05.05.2022 Gazette 2022/18)**

(54) **TRANSFORMED IMMUNE CELLS INDUCING CHEMOTAXIS TOWARDS HETEROGENEOUS IMMUNE CELLS**

TRANSFORMIERTE IMMUNZELLEN ZUR INDUZIERUNG VON CHEMOTAXIS GEGEN HETEROGENE IMMUNZELLEN

CELLULES IMMUNITAIRES TRANSFORMÉES INDUISANT LA CHIMIOTAXIE VERS DES CELLULES IMMUNITAIRES HÉTÉROGÈNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2020 KR 20200141039**

(43) Date of publication of application:
**06.09.2023 Bulletin 2023/36**

(73) Proprietor: **TSD Life Sciences Co., Ltd.**
**Seoul 06159 (KR)**

(72) Inventors:
• **CHOI, Ki Doo**
  **Suwon-si Gyeonggi-do 16956 (KR)**
• **SON, Dohyun**
  **Namyangju-si Gyeonggi-do 12275 (KR)**
• **LEE, Milim**
  **Goyang-si Gyeonggi-do 10498 (KR)**
• **OH, Sanghyeon**
  **Seoul 04338 (KR)**

(74) Representative: **Müller, Christian Stefan Gerd**
**ZSP Patentanwälte PartG mbB**
**Hansastraße 32**
**80686 München (DE)**

(56) References cited:
  WO-A1-2019/073973    WO-A1-2019/124468
  WO-A1-2020/027832    WO-A1-2021/085497
  WO-A1-2021/244654    WO-A1-2022/227511
  WO-A1-2023/007431    CN-A- 108 949 789
  CN-A- 110 590 960    CN-C- 100 359 004
  KR-A- 20190 051 039    US-A1- 2014 255 363

• **DUAN DEMING ET AL: "The BCMA-Targeted Fourth-Generation CAR-T Cells Secreting IL-7 and CCL19 for Therapy of Refractory/Recurrent Multiple Myeloma", vol. 12, 5 March 2021 (2021-03-05), XP093071267, Retrieved from the Internet <URL:https://www.ncbi.nlm.nih.gov/pmc/articles/PMC7985831/pdf/fimmu-12-609421.pdf> [retrieved on 20240912], DOI: 10.3389/fimmu.2021.609421**

EP 4 239 060 B1

- PANG NENGZHI ET AL: "IL-7 and CCL19-secreting CAR-T cell therapy for tumors with positive glypican-3 or mesothelin", vol. 14, no. 1, 29 July 2021 (2021-07-29), XP093058245, Retrieved from the Internet <URL:https://link.springer.com/article/10.1186/s13045-021-01128-9/fulltext.html> [retrieved on 20240912], DOI: 10.1186/s13045-021-01128-9
- ADACHI KEISHI ET AL: "The novel strategies for CAR-T cell therapy targeting solid cancers", EXPERIMENTAL MEDICINE, vol. 38, no. 17, Suppl. (665), 1 November 2020 (2020-11-01), JP, XP093041413, ISSN: 0288-5514
- GHAHRI-SAREMI NAVID ET AL: "Genetic Modification of Cytokine Signaling to Enhance Efficacy of CAR T Cell Therapy in Solid Tumors", FRONTIERS IN IMMUNOLOGY, vol. 12, 14 October 2021 (2021-10-14), XP093089785, DOI: 10.3389/fimmu.2021.738456
- ADACHI, K. ET AL.: "IL -7 and CCL19 expression in CAR-T cells improves immune cell infiltration and CAR-T cell survival in the tumor", NATURE BIOTECHNOLOGY, vol. 36, no. 4, 2018, pages 346 - 351, XP055474269, DOI: 10.1038/nbt.4086
- LUO, H. ET AL.: "Coexpression of IL 7 and CCL21 Increases Efficacy of CAR-T Cells in Solid Tumors without Requiring Preconditioned Lymphodepletion", CLIN. CANCER RES., vol. 26, no. 20, 15 October 2020 (2020-10-15), pages 5494 - 5505, XP055921328, DOI: 10.1158/1078-0432.CCR-20-0777

Remarks:
•The complete document including Reference Table(s) and the Sequence Listing(s) can be downloaded from the EPO website
•The file contains technical information submitted after the application was filed and not included in this specification

## Description

### TECHNICAL FIELD

The present invention relates to natural killer cells, that express specific chemotactic factors for the migration of T cells to the lesion site.

### BACKGROUND OF THE INVENTION

Cancer is basically a disease associated with abnormal growth regulation of tissues, and cancer cells grow and proliferate excessively compared to normal cells and characteristically invade adjacent tissues or metastasize to distant tissues. Anti-cancer treatment inhibits the expression of genes necessary for cancer cell survival, such as oncogenes, telomerase, growth factor receptors, or signaling molecules, or induces cell death through physical removal of cancer tissue, irradiation, or administration of anticancer drugs. However, since normal cells may be also damaged alongside cancer cells during this practice, immune cell therapy that specifically remove tumor tissue are receiving attention. In particular, anti-cancer therapy that remove tumor tissue by activating the patient's bodily immune response using immune cells such as dendritic cells, natural killer cells and T cells have been intensively studied in the past decades.

Natural killer cells (NK cells) are lymphoid cells that account for about 15% of peripheral blood lymphocytes and play a crucial role in the innate immune response. In particular, they remove tumor cells by activating dendritic cells and inducing cytotoxic T lymphocytes (CTLs) to specifically react to the tumor. Natural killer cells directly kill malignant tumors including sarcomas, myelomas, lymphomas and leukemia, and its therapeutic effect was confirmed by administration of *in vitro* - activated NK cells to patients with blood cancer, such as leukemia, after bone marrow transplantation (Blood Cells Molecules & Disease, 33: p261-266, 2004). However, since most natural killer cells are in an inactive state in the body of a normal person, studies have been focused on *in vitro* activation of NK cells isolated from blood for increased treatment efficiency.

On the other hand, T cells, which constitute another axis among immune cells, are lymphocytes that are generated in the bone marrow and matured in the thymus. They have memory functions in the immune system and provide information to B cells to induce antibody production. After undergoing an immune tolerance test in the thymus, T cells differentiate into 4 types: cytotoxic T cells, helper T cells, regulatory T cells and memory T cells. Cytotoxic T cells, which are CD8+, bind to type I MHC to identify cancer cells or infected cells, and mainly target lesional cells like natural killer cells.

Although therapeutic effects can be maximized when natural killer cells and cytotoxic T cells are administered in combination, the development of novel therapies that can overcome the inefficiency of injecting two cells with each therapeutically effective amount is required.

CN 100 359 004 C discloses natural killer cells expressing CCL19 to enhance the *in vivo* bioavailability of these natural killer cells.

Throughout this application, various publications and patents are referred and citations are provided in parentheses, in order to fully describe this invention and the state of the art to which this invention pertains.

### SUMMARY OF THE PRESENT INVENTION

The present invention is defined by the appended claims.

In a first aspect, the present invention provides a natural killer cell artificially expressing IL-7 (interleukin-7) and CCL19 (C-C Motif Chemokine Ligand 19).

In a second aspect, the present invention provides a composition for use in a method of preventing or treating cancer or infectious diseases comprising the natural killer cell according to the first aspect as an active ingredient.

### DISCLOSURE

### TECHNICAL PROBLEM

The present inventors have made intensive studies to develop an excellent cell therapy enabling effective removal of diseased cells or tissues through a complex immune response by a combination of heterogeneous immune cells. As results, the present inventors have discovered that when immune cells, specifically immune cells other than T cells, and more specifically natural killer cells, that express IL-7, CCL19 or a combination thereof, are injected into a subject, endogenous T cells proliferated and homed by the IL-7 and CCL19, along with injected natural killer cells, simultaneously act on the lesion site and thus induce a multifaceted and synergistic immune response.

Other objectives and advantages of the present invention will become apparent from the following detailed description together with the appended claims and drawings.

## TECHNICAL SOLUTION

Described herein is an immune cell expressing a nucleic acid molecule encoding IL-7 (interleukin-7) or a functional portion thereof, a nucleic acid molecule encoding CCL19 (C-C Motif Chemokine Ligand 19) or a functional portion thereof, or a combination thereof.

The present inventors have made intensive studies to develop an excellent cell therapy enabling effective removal of diseased cells or tissues through a complex immune response by a combination of heterogeneous immune cells. As results, the present inventors have discovered that when natural killer cells, that express IL-7 and CCL19, are injected into a subject, endogenous T cells proliferated and homed by the IL-7 and CCL19, along with injected natural killer cells, simultaneously act on the lesion site and thus induce a multifaceted and synergistic immune response. Accordingly, in a first aspect of the invention, there is provided a natural killer cell artificially expressing IL-7 (interleukin-7) and CCL19 (C-C Motif Chemokine Ligand 19).

The term "immune cell" as used herein refers to any cell involved in the initiation or promotion of an immune response, and more particularly, an immune effector cells. Immune cells include, for example, T cells, B cells, natural killer (NK) cells, natural killer T (NKT) cells, mast cells and dendritic cells, but are not limited thereto. More concretely, the immune cell is an immune cell other than T cell, and most concretely, a natural killer cell.

The term "T cell" as used herein is a group of cells in the same scope as those understood in the art and includes CD8+ or CD4+ T cells that directly lyses target cells or provides an effector function or helper function which causes the death of target cells, but is not limited thereto and may includes any cell classified as "T cell" in the art to which the present invention pertains.

The term "functional portion" as used herein refers to an equivalent fragment of full-length protein form where certain amino acid residues are deleted, which maintains original biological activity and function of the full-length protein.

The term "nucleic acid molecule" as used herein has comprehensive meaning including DNA (gDNA and cDNA) and RNA molecule. A nucleotide, which is a basic construct unit of nucleic acid molecule, includes nucleotide analogues with modified sugar or base, as well as natural-occurring nucleotides (Scheit, Nucleotide Analogs, John Wiley, New York (1980); Uhlman and Peyman, Chemical Reviews, 90:543-584(1990)).

For nucleotides, the variations may be purely genetic, i.e., ones that do not result in changes in the protein product. This includes nucleic acids that contain functionally equivalent codons, or codons that encode the same amino acid, such as six codons for arginine or serine, or codons that encode biologically equivalent amino acids. Considering biologically equivalent variations described hereinabove, the nucleic acid molecule of this disclosure may encompass sequences having substantial identity to them. Sequences having the substantial identity show at least 80%, concretely at least 85%, more concretely at least 90%, and most concretely at least 95% similarity to the nucleic acid molecule of this disclosure, as measured using one of the sequence comparison algorithms well-known in the art.

The term "express" as used herein includes artificial expression of an exogenous gene that is not naturally expressed by the immune cell described herein through a gene carrier, natural expression of an endogenous gene through an endogenous expression system, and overexpression of endogenous gene by increasing expression of endogenous genes by a gene carrier. Therefore, the immune cell described herein includes a cell endogenously expressing IL-7 and CCL19, a cell endogenously expressing IL-7 and artificially expressing CCL19, a cell endogenously expressing CCL19 and artificially expressing IL-7, and a cell artificially expressing IL-7 and CCL19.

The term "to express" as used herein refers to being artificially replicated as an extrachromosomal factor or by chromosomal integration in a target cell via a gene delivery system to cause the target cell to express a foreign gene or overexpress an endogenous gene. Accordingly, "express" in the term "to express" may be used interchangeably with "transformation", "transfection", or "transduction".

The "gene delivery system" as used herein refers to any means of delivering a gene into a cell. The gene delivery has the same meaning as intracellular transduction of genes. At the tissue level, the term gene delivery has the same meaning as the spread of a gene. Accordingly, the gene delivery system can be described as a gene penetration system or a gene spread system.

The nucleotide sequences of the IL-7 and CCL19 gene may be applied to all gene delivery systems used for conventional gene transfers, such as plasmid, adenovirus, adeno-associated virus, retrovirus, lentivirus, herpes simplex virus, vaccinia virus, liposome or niosome.

Where the gene delivery system is a naked recombinant DNA molecule or a plasmid, the gene can be introduced into cells by microinjection (Capecchi, M.R., Cell, 22:479(1980)), calcium phosphate precipitation (Graham, F.L. et al., Virology, 52:456(1973)), electroporation (Tur-Kaspa et al., Mol. Cell Biol., 6:716-718(1986)), liposome-mediated transfection (Biochim. Biophys. Acta, 721:185-190(1982)), DEAE-dextran treatment (Gopal, Mol. Cell Biol., 5:1188-1190(1985)) and gene bombardment (Yang et al., Proc. Natl. Acad. Sci., 87:9568-9572(1990)), and concretely, electroporation may be used.

According to a concrete embodiment, the IL-7 comprises the amino acid sequences with at least 85% sequence similarity, more concretely at least 90% similarity, and most concretely at least 95% sequence similarity with the amino acid sequence of SEQ ID NO:1.

According to a concrete embodiment, the CCL19 comprises amino acid sequences with at least 85% or more similarity, more concretely at least 90% or more sequence similarity, and most concretely at least 95% or more sequence similarity with the amino acid sequence of SEQ ID NO:2.

According to a concrete embodiment, nucleic acid molecules encoding IL-7 or a functional portion thereof comprises the nucleotide sequence of SEQ ID NO:3.

According to a concrete embodiment of the present invention, nucleic acid molecules encoding CCL19 or a functional portion thereof comprises the nucleotide sequence of SEQ ID NO:4.

According to the present invention, SEQ ID NO:3 and SEQ ID NO:4 are codon optimized nucleotide sequences of IL-7 and CCL19, respectively, for effective expression in natural killer cells.

Further described herein is a composition for the prevention or treatment of cancer or infectious diseases comprising the aforementioned immune cells described herein as an active ingredient. The present invention, in a second aspect, provides a composition for use in a method of preventing or treating cancer or infectious diseases comprising the natural killer cell of the first aspect as an active ingredient.

In case the natural killer cells of the present invention are applied as cell therapy, it may be utilized for the treatment of various tumors and infectious diseases. The natural killer cells of the present invention can be applied to all types of tumors including solid cancer and blood cancer. Such cancer may include, but not limited thereto, gastric cancer, liver cancer, lung cancer, colorectal cancer, breast cancer, prostate cancer, ovarian cancer, pancreatic cancer, cervical cancer, thyroid cancer, laryngeal cancer, acute myelogenous leukemia, brain tumor, neuroblastoma, retinoblastoma, head and neck cancer, salivary gland cancer and lymphoma. Infectious diseases that may be prevented or treated by the natural killer cells of the present invention may be diseases caused by viral or pathogenic infections, which include all diseases that can be infected through respiratory, blood, skin contact, and the like. Such infectious disease include, but are not limited to, hepatitis B and C, human papilloma virus (HPV) infection, cytomegalovirus infection, viral respiratory disease and influenza.

The term "prevention" as used herein refers to suppressing the outbreak of illness or disease in a subject who has not been diagnosed with the illness or disease, but is likely to suffer from the illness or disease.

The "treatment" as used herein refers to (a) inhibition of the development of a disease, illness or symptoms, (b) alleviation of the disease, illness or symptoms, or (c) elimination of the disease, illness or symptoms. When the natural killer cells of the present invention are administered to a subject, a complex immune response is generated by natural killer cells and endogenous T cells or exogenously injected autologous or allogeneic T cells recruited to the lesion site by the natural killer cells, which serves to inhibit, eliminate or alleviate the development of symptoms caused by tumors or infectious diseases by inducing the killing of cancer cells, infected cells, or pathogens. Therefore, the composition described herein may itself be a cell therapy composition for diseases, or may be applied as a therapeutic adjuvant for the disease by being administered together with other active ingredients, such as therapeutic T cells or other known anticancer agents. Accordingly, the term "treatment" or "therapeutic agent" in the present specification includes the meaning of "therapeutic aid" or "therapeutic adjuvant".

The term "administration" or "to administer" as used herein refers to the direct administration of a therapeutically effective amount of the composition of the present invention to a subject so that the same amount is formed in the body of the subject.

The terms "therapeutically effective amount" as used herein refer to the content of the composition of the present invention that is sufficient to provide a therapeutic or prophylactic effect to a subject to whom the composition is to be administered, and thus include the meaning of a "prophylactically effective amount".

The term "subject" as used herein includes, without limitation, humans, mice, rats, guinea pigs, dogs, cats, horses, cows, pigs, monkeys, chimpanzees, baboons or rhesus monkeys. Concretely, the subject of the present invention is humans.

Further described herein is a composition for inducing proliferation or homing of a heterogeneous immune cell comprising a nucleic acid molecule encoding IL-7 (interleukin-7) or a functional portion thereof, a nucleic acid molecule encoding CCL19 (C-C Motif Chemokine Ligand 19) or a functional portion thereof, or a combination thereof, as an active ingredient.

Since the nucleic acid molecules to be used have already been described in detail above, descriptions thereof are omitted to avoid excessive redundancy.

The term "heterogeneous immune cells" used herein refers to immune cells of a different type from the target cell into which the nucleic acid molecule is introduced. Concretely, the heterogeneous immune cells are T cells or dendritic cells. A large amount of endogenous T cells can be migrated to the lesion by injecting immune cells simultaneously expressing IL-7 and CCL19, concretely immune cells other than T cells, and most concretely natural killer cells, into the subject to induce proliferation and chemotaxis of T cells. The term "chemotaxis" refers to the positive (towards a stimulus) or negative (away from a stimulus) migration of cells which is induced by chemical stimuli, and more concretely refer to positive migration. T cells are proliferated and differentiated by IL-7 and activated by CCL19, a corresponding chemotactic factor, and then move toward natural killer cells expressing IL-7 and CCL19. This results in formation of heterogeneous cell population composed of two types of complementary immune cells, i.e. T cells and natural killer cells, around the lesion site. Accordingly, the composition described herein may act as a therapeutic adjuvant that provides the effect for co-administration of different cell populations only by a therapeutically effective amount of a single cell type.

According to a concrete embodiment, the nucleic acid molecules can be included in the composition by being inserted together into single gene delivery system or each separately into two different gene delivery systems.

Further described herein is a method for preventing or treating cancer or infectious diseases comprising administering the aforementioned immune cells to a subject. Since the immune cells to be used and the cancer and infectious diseases that can be prevented or treated thereby have already been described above in detail, descriptions thereof are omitted to avoid excessive redundancy.

Further described herein is a method for inducing a proliferation or homing of a heterogeneous immune cell comprising introducing a nucleic acid molecule encoding IL-7 (interleukin-7) or a functional portion thereof, a nucleic acid molecule encoding CCL19 (C-C Motif Chemokine Ligand 19) or a functional portion thereof, or a combination thereof, into an immune cell. Since the nucleic acid molecules and heterologous immune cells to be used have already been described above in detail, descriptions thereof are omitted to avoid excessive redundancy.

## EFFECTS OF THE INVENTION

The features and advantages of the present invention are summarized as follows:

(a) The present invention provides natural killer cells expressing IL-7 and CCL19 thereof, and a composition for the preventing or treating cancer or infectious diseases comprising the same as an active ingredient.
(b) The present invention may achieve a multifaceted and synergistic therapeutic effect through administering a therapeutically effective amount of natural killer cells, due to the complementary immune responses between the patient's endogenous T cells and injected natural killer cells.
(c) According to the present invention, co-administration of T cells and the natural killer cells, also significantly improve therapeutic effects by allowing these heterogeneous cell populations to act in a lesion-concentrated manner.

## BRIEF DESCRIPTION OF THE DRAWINGS

Figure 1 represents a schematic diagram showing the structure of the pEF1-IRES empty vector used as a negative control vector.
Figure 2 represents a schematic diagram showing the structure of the pEF1-IRES vector into which IL-7 and CCL19 genes are inserted.
Figure 3 represents a schematic diagram showing the structure of the pEF1-IRES vector into which CCL19 and IL-7 genes are inserted.

Hereinafter, the present invention will be described in further detail by examples. It would be obvious to those skilled in the art that these examples are intended to be more concretely illustrative and the scope of the present invention as set forth in the appended claims is not limited to or by the examples.

## Examples

### Example 1: Construction of IL-7 and CCL19 Expression Vectors

Expression vectors were constructed to prepare human NK cells expressing IL-7 and CCL19. A pEF1$\alpha$-IRES bicistronic mammalian expression vector (Takara, CAT#631970) under the control of human elongation factor 1 alpha (EF1$\alpha$) promoter was used as an expression vector.

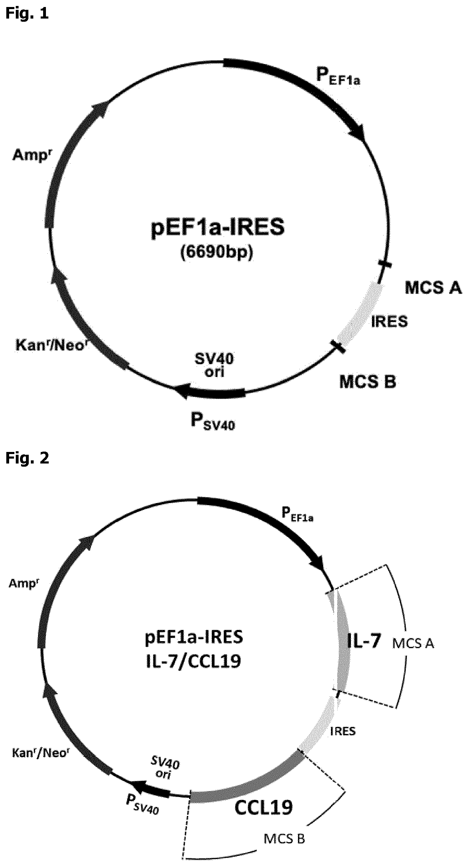

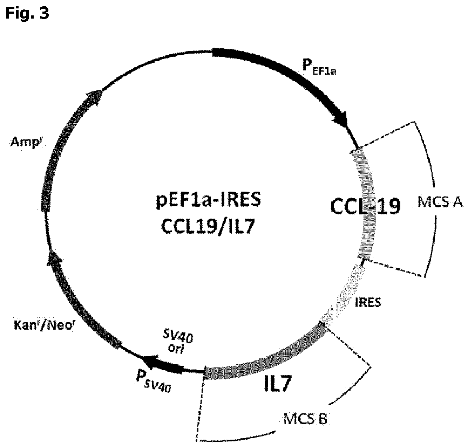

The ORF sequences of IL-7 and/or CCL19 genes to be inserted into the vector were codon-optimized (SEQ ID NO:3 and SEQ ID NO:4, respectively) and synthesized. IL-7 or CCL19 was cloned into the multicloning site A (MCS A) of the pEF1$\alpha$-IRES vector, and CCL19 or IL-7 was cloned into MCS B. Here, an internal ribosome entry site (IRES) was positioned between the two MCSs in the pEF1$\alpha$-IRES so that the two genes could be co-expressed. The structure of the empty vector, the negative control, is shown in Figure 1, the structure of the IL-7/CCL19 expression vector is shown in Figure 2, and the structure of the CCL19/IL-7 expression vector is shown in Figure 3, respectively.

**Example 2: Confirmation of the Expression of IL-7 and CCL19**

The NK92 cell line was purchased from ATCC (CAT#CRL-2407), and cultured in α-MEM (Alpha Minimum Essential Medium) containing 12.5% horse serum (Sigma, CAT#H1270), 12.5% fetal bovine serum (FBS, Gibco, CAT#10099141), and interleukin-2 (IL-2, Peprotech, CAT#200-02). In order to prepare a NK92 cell line expressing IL-7 and CCL19 (hereinafter, "transformed NK92 cell line"), electroporation was performed at a density of $1 \times 10^6$ NK92 cells/0.1mL.

The electroporation was performed by NeonTM Transfection System (ThermoFisher, CAT#MPK5000) was used under the following conditions: 1800-1850 v voltage, 10ms pulse length, 1 time, and 10-20 μg DNA (negative control vector, IL-7/CCL19 expression vector, or CCL19/IL-7 expression vector prepared in Example 1). After culturing for 1 day in 2mL of RPMI1640 medium (Gibco #11875168) containing 10% FBS, the expression level of IL-7 or CCL19 was measured or a transwell assay were performed.

In order to measure the expression level of IL-7 and CCL19 in the transformed NK92 cell line, the culture medium was collected in a 6-well plate. After centrifugation at 800xg for 5 minutes, the supernatant was separated. 0.1mL per well of the supernatant was appropriately diluted and used for IL-7 or CCL19 enzyme linked immunosorbent assay (ELISA; IL-7, Komabiotech, CAT#k0331215; CCL19, Abcam, CAT#ab100601) to measure the expression level of IL-7 or CCL19.

The ELISA for IL-7 and CCL19 was performed according to the standard testing methods of each manufacturer. The final values measured at a wavelength of 450 nm are shown in Table 1.

[Table 1]

| Amount of IL-7 and CCL19 secretion in transformed cells | | | |
|---|---|---|---|
| **Cell** | **Gene** | **IL-7 (pg/mL)** | **CCL19 (pg/mL)** |
| NK92 | Empty vector | N.D | 4.2 |
| | IL-7/CCL19 | 1,656 | 242 |
| | CCL19/IL-7 | 157 | 368 |
| ＊ N.D : Not Detected | | | |

The results of Table 1 indicate that the IL-7/CCL19 NK92 cell line and the CCL19/IL-7 NK92 cell line secreted IL-7 and CCL19, while IL-7 and CCL19 were not detected or were detected at insignificant levels in the negative control cells injected with the empty vector.

**Example 3: Analysis of T cell chemotaxis and proliferation induced by IL-7 and CCL19 expressing NK Cells**

To confirm the effect of the secretion of IL-7 and CCL19 by NK cells on T cell chemotaxis, a 12-well transwell (Corning, CAT#CLS3421) was used. 5μm polycarbonate was used as the chamber filter.

Comparison of IL-7/CCL19 NK92 cells and negative controls using HuT78 T cell line

$1 \times 10^7$ cells of HuT78 (ATT, CAT#TIB-161), the T cells, were cultured for 24 hours in IMDM (Gibco, CAT#12440053) medium containing 1% PS without FBS. Thereafter, 100μl of HuT78 cells at a density of $5 \times 10^6$ cells/mL were dispensed into the upper chamber using the same medium as above, and the culture solution cultured for 2-3 days in the IL-7/CCL19 NK92 cell line prepared in Example 2 was dispensed in the lower chamber.

After culturing for 1 day in an incubator at 37°C and 5% $CO_2$, the upper chamber was removed and further cultured for 3 days. To measure the number of cells in the lower chamber, 400μl of medium per well was centrifuged at 800xg for 5 minutes to remove the supernatant and obtain a concentrated sample. 10μl of Trypan blue was mixed with 10μl of the concentrated sample at a 1:1 ratio and measured with Countess™II (Invitrogen, CAT#AMQAX1000). The results were converted into the total number of viable cells in 400μl as shown in Table 2.

[Table 2]

| Chemotaxis of the HuT78 T cell line by transformed NK cells | |
|---|---|
| **Experimental Group** | **Number of cells in the lower chamber (mean $\pm$ standard deviation)** |
| Negative control cells | 3,836±276 |

(continued)

| Chemotaxis of the HuT78 T cell line by transformed NK cells | |
|---|---|
| **Experimental Group** | **Number of cells in the lower chamber (mean $\pm$ standard deviation)** |
| IL-7/CCL19 NK92 cells | 11,989$\pm$887 |

Comparison of IL-7/CCL19 NK92 cells, CCL19/IL-7 NK92 cells and negative control using PBMC-derived T cells

To evaluate the chemotaxis of peripheral blood mononuclear cells (PBMC)-derived T cells, T cells were isolated from PBMC using the EasySepTM Human T Cell Isolation Kit (STEMCELL #17951). After diluting the T cells at a concentration of $2 \times 10^6$ cells/mL with RPMI1640 medium containing 2% FBS, 100$\mu$l was dispensed into the upper chamber. In the lower chamber, the culture solution cultured for 1 day in IL-7/CCL19 and CCL19/IL-7 transformed NK92 cell lines prepared in Example 2 was dispensed.

After culturing for 1 day in an incubator at 37°C and 5% $CO_2$, the upper chamber was removed and further cultured for 3 days. To measure the number of cells in the lower chamber, 500$\mu$l of medium per well was centrifuged at 800xg for 5 minutes to remove the supernatant and obtain a concentrated sample. 10$\mu$l of Trypan blue was mixed with 10$\mu$l of the concentrated sample at a 1:1 ratio and measured with Countess™II. The results were converted into the total cell numbers as shown in Table 3.

**[Table 3]**

| PBMC-derived T cell chemotaxis by transformed NK92 cell line | | |
|---|---|---|
| **T cell type** | **Experimental group (n=2)** | **Number of T cells migrating to lower chamber (mean $\pm$ standard deviation, $10^4$ cells)** |
| PBMC-derived T cells | Negative control NK92 cells | 1.89 $\pm$ 0.75 |
| | IL-7/CCL19 NK92 cells | 9.35 $\pm$ 0.53 |
| | CCL19/IL-7 NK92 cells | 6.46 $\pm$ 0.16 |

As shown in Tables 2 and 3, the number of T cells that migrated to the lower chamber was significantly higher in the IL-7/CCL19 and CCL19/IL-7 NK92 cell line group compared to the negative control empty vector-introduced NK92 cell line. This indicates that the NK92 cell transformed with IL-7 and CCL19 induced chemotaxis of T cells and proliferation of migrated T cells.

**Example 4: Measurement of cancer cell killing and secretion of the related factors by transformed NK92 cell lines and T cells migrated thereby**

After HepG2 liver cancer cell line (ATCC #HB-8065) was diluted in RPMI1640 medium containing 10% FBS to a concentration of $2 \times 10^5$ cells/mL, 0.1mL was dispensed into each well of a 96-well plate and cultured for 1 day. T cell migration was induced for 1 day by transferring 0.5mL RPMI1640 medium containing the transformed NK92 cell line cultured for 1 day by electroporation in the same manner as in Example 2 to the lower chamber of the transwell in the same manner as in Example 3. $2 \times 10^5$ cells/0.1mL of T cells isolated from PBMC were transferred to the upper chamber. Here, $1 \times 10^5$ CD3/CD28 Dynabeads and 100U IL-2 were added to the lower chamber to activate the migrating T cells. After culturing for 1 day, the upper chamber was removed and transferred 0.1mL per well to the 96-well plate in which the HepG2 liver cancer cell line was being cultured. After culturing for 3 more days and washing three times using PBS (phosphate buffer saline), the medium was replaced with 0.1mL of 10% FBS-RPMI1640 medium containing 1% of CCK-8 and further cultured for 2 hours. In order to evaluate the cell viability of the HepG2 liver cancer cell line, absorbance was measured at 450nm and the degree of cell viability was calculated using [Equation 1], as shown in Table 4.

[Equation 1]

$$\text{HepG2 cell line viability} = 100 \times \frac{(\text{Test group absorbance value} - \text{RPMI1640 medium absorbance value})}{(\text{Normal group absorbance value} - \text{RPMI1640 medium absorbane value})}$$

In addition, the secretion of granzyme-B (Grz-B), interferon-$\gamma$ (IFN-$\gamma$), and tumor necrosis factor-$\alpha$ (TNF-$\alpha$) from activated T cells and activated NK cells was measured using the medium collected from the cell viability measurement above, via the enzyme-linked immunosorbent assay (ELISA; GrzB, abcam #ab235635; IFN-γ, komabiotech #K0331121; TNF-α, komabiotech #K0331131), of which the results are shown in Table 5. Each value in Table 4 and 5 was expressed as mean ± standard deviation.

[Table 4]

| Cytotoxicity for HepG2 liver cancer cell line | | |
|---|---|---|
| Cancer cell line | Experimental group (n=3) | Cell viability |
| HepG2 | Medium (Normal group) | 100.0 ± 7.8 |
| | Negative control group (Empty vector NK92) | 83.8 ± 2.1 |
| | IL-7/CCL19 NK92 cells | 33.2 ± 3.3 |
| | CCL19/IL-7 NK92 cells | 52.2 ± 3.5 |

[Table 5]

| Secretion Grz-B, IFN-γ and TNF-α | | | | |
|---|---|---|---|---|
| Cancer cell line | Experimental group (n=2) | Grz-B (pg/mL) | IFN-γ (pg/mL) | TNF-α (pg/mL) |
| HepG2 | Medium (Normal group) | 592 ± 179 | N.D | N.D |
| | Negative control (Empty vector NK92) | 1,259 ± 81 | 8±1 | N.D |
| | IL-7/CCL19 NK92 cells | 2,573 ± 450 | 320 ± 28 | 195 ± 37 |
| | CCL19/IL-7 NK92 cells | 1,460 ± 466 | 71 ± 3 | 33 ± 10 |
| ✳ N.D : Not Detected | | | | |

As shown in Table 4, significantly higher cell killing activity toward HepG2 was observed in the experimental group of IL-7/CCL19 and CCL19/IL-7 NK92 cell line compared to the empty vector-introduced NK92 cell as a negative control. As shown in Table 5, the factors related to the cytotoxicity for HepG2 cell line also showed higher secretion in the groups of IL-7/CCL19 and CCL19/IL-7 NK92 cell line compared to the negative control group. Accordingly, it was confirmed that both the IL-7/CCL19 NK cell line and the CCL19/IL-7 NK cell line may be applied as an effective anti-cancer treatment.

## Claims

1. A natural killer cell artificially expressing IL-7 (interleukin-7) and CCL19 (C-C Motif Chemokine Ligand 19).

2. A composition for use in a method of preventing or treating cancer or infectious diseases comprising the natural killer cell according to claim 1 as an active ingredient.

## Patentansprüche

1. Natürliche Killerzelle, die IL-7 (Interleukin-7) und CCL19 (C-C-Motiv-Chemokin-Ligand 19) künstlich exprimiert.

2. Zusammensetzung zur Verwendung in einem Verfahren zum Verhindern oder Behandeln von Krebs oder Infektionskrankheiten, umfassend die natürliche Killerzelle gemäß Anspruch 1 als einen aktiven Bestandteil.

## Revendications

1. Cellule tueuse naturelle exprimant artificiellement l'IL-7 (interleukine-7) et le CCL19 (ligand 19 de chimiokine à motif C-C).

2. Composition destinée à être utilisée dans un procédé de prévention ou de traitement du cancer ou de maladies infectieuses comprenant la cellule tueuse naturelle selon la revendication 1 en tant que principe actif.

**Fig. 1**

**Fig. 2**

**Fig. 3**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 100359004 C

**Non-patent literature cited in the description**

- *Blood Cells Molecules & Disease*, 2004, vol. 33, 261-266
- **SCHEIT**. Nucleotide Analogs. John Wiley, 1980
- **UHLMAN** ; **PEYMAN**. Chemical Reviews. 1990, vol. 90, 543-584
- **CAPECCHI, M.R.** *Cell*, 1980, vol. 22, 479
- **GRAHAM, F.L. et al.** *Virology*, 1973, vol. 52, 456
- **TUR-KASPA et al.** *Mol. Cell Biol.*, 1986, vol. 6, 716-718
- *Biochim. Biophys. Acta*, 1982, vol. 721, 185-190
- *Gopal, Mol. Cell Biol.*, 1985, vol. 5, 1188-1190
- **YANG et al.** *Proc. Natl. Acad. Sci.*, 1990, vol. 87, 9568-9572